# EUROPÄISCHE PATENTSCHRIFT

(11) **EP 0 445 560 B1**
(45) Veröffentlichungstag und Bekanntmachung des Hinweises auf die Patenterteilung: **27.09.1995**
(21) Anmeldenummer: 91102235.8
(22) Anmeldetag: 18.02.1991
(51) Int. Cl.: C07C 19/08, C07C 17/20, C07C 17/00

(54) **Verfahren zur Herstellung von Äthanderivaten**
Process for preparing ethane derivatives
Procédé pour la préparation de dérivés d'éthane

(30) Priorität: 26.02.1990 DE 4005945
(43) Veröffentlichungstag der Anmeldung: 11.09.1991
(73) Patentinhaber: Kali-Chemie Aktiengesellschaft, D-30173 Hannover (DE)
(72) Erfinder: Fernschild, Günther, verstorben (DE); Rudolph, Werner, W-3000 Hannover 71 (DE); Brosch, Carsten, W-3016 Seelze 8 (DE)
(74) Vertreter: Lauer, Dieter, Dr.

(56) Entgegenhaltungen:
- EP-A- 0 282 005
- EP-A- 0 300 724
- EP-A- 0 414 370
- WO-A-90/01474
- DE-B- 1 083 247
- US-A- 2 510 872
- US-A- 4 078 007
- Berichte der Deutsche Chemie Gesellschaft 37(1904), s.673-683

## Beschreibung

Die Erfindung betrifft ein Verfahren zur Herstellung von Trifluormethylgruppen-haltigen Äthanderivaten der allgemeinen Formel CF₃CHYZ (I), wobei Y und Z gleich oder verschieden sein können und Wasserstoff, Chlor oder Brom oder Fluor bedeuten.

Perhalogenierte organische Verbindungen, die als Kühlmittel, als Entfettungsmittel, als Schmier- und Trennmittel oder bei der Herstellung geschäumter Kunststoffe verwendet werden, werden zunehmend durch wasserstoffhaltige halogenierte organische Verbindungen ersetzt.

Eine sehr gut als Ersatz für perhalogenierte organische Verbindungen geeignete Stoffklasse sind Trifluormethylgruppen-haltige Äthanderivate der allgomeinen Formel CF₃CHYZ (I). Y und Z können gleich oder verschieden sein und Wasserstoff, Fluor, Chlor oder Brom bedeuten.

Verbindungen dieser Art können durch Halogen-Fluor-Austausch hergestellt werden. wobei es sich üblicherweise um einen Chlor-Fluor-Austausch handelt. Ein Nachteil bekannter Herstellungsverfahren ist die schlechte Ausbeute. Ferner entstehen häufig auch perhalogenierte Nebenprodukte, die unerwünscht sind.

Die technische Herstellung dieser Verbindungen ist jedoch mit Schwierigkeiten verbunden. Dies liegt daran, daß höher halogenierte Verbindungen gegenüber chemischen Reaktionen verhältnismäßig inert sind.

Es sind Verfahren bekannt, die die Herstellung von CF₃CHCl₂ zum Ziel haben. Eine Herstellung von CF₃CHCl₂ in der Flüssigphase wird in der Veröffentlichung von A.E. Feiring in J. Fluor. Chem. 13 (1979), Seiten 7 bis 18, beschrieben. Dort wird Fluortetrachlorethan mit Fluorwasserstoff in Anwesenheit von Tantalpentafluorid als Katalysator in der Flüssigphase umgesetzt. Die Ausbeute an CF₃CHCl₂ beträgt etwa 10 %. Ausgehend von Tetrachlorethylen gelingt die Herstellung nur mit einem einzigen Katalysator, nämlich wiederum mit Tantalpentafluorid, allerdings in einer Ausbeute von nur 2 %.

Die deutsche Offenlegungsschrift DE-A 1 083 247 offenbart ein Verfahren zur Herstellung von CF₂ClCHBrCl und CF₃CHBrCl durch Umsetzung von CCl₃CHBrCl mit HF unter Druck. Man arbeitet in Gegenwart eines Antimonfluoridchloridkatalysators mit mindestens 20% fünfwertigem Antimon.

Die europäische Patentanmeldung EP-0 424 531 A1 offenbart die Herstellung von 1,1-Dichlor-2,2,2-trifluorethan aus 1,1,2-Trichlor-2,2-difluorethan und/oder 1,1,2,2-Tetrachloro-1-fluorethan mit HF in flüssiger Phase in Anwesenheit von Antimonpentachlorid. Die Ausgangsverbindungen können ihrerseits durch Flüssigphasenfluorierung von Trichlorethylen oder Tetrachlorethylen hergestellt werden. Die Autoren gehen davon aus, daß Antimonpentachlorid in Anwesenheit von HF teilweise fluoriert wird.

Ein in der Gasphase durchgeführtes Verfahren, das als einziges technisch brauchbares Verfahren zur Herstellung von CF₃CHCl₂ bezeichnet wird, wird in der EP-A 282 005 beschrieben. Die Herstellung von CF₃CHCl₂ erfolgt bei Temperaturen von 320 bis 360 °C durch Umsetzung von Tetrachlorethylen mit Fluorwasserstoff über Aluminiumfluorid/Chromoxid-Katalysatoren. Umwandlungsgrad und Ausbeute in diesem Verfahren sind unbefriedigend, der Energieverbrauch naturgemäß sehr hoch.

Aufgabe der Erfindung ist es, ein verbessertes Verfahren zur Herstellung von Äthanderivaten der allgemeinen Formel CF₃CHYZ (I) in flüssiger Phase anzugeben. Diese Aufgabe wird durch das erfindungsgemäße Verfahren gelöst.

Das erfindungsgemäße Verfahren zur Herstellung von die Trifluormethylgruppe enthaltenden Äthanderivaten durch Halogen-Fluoraustausch mit im wesentlichen wasserfreiem Fluorwasserstoff in flüssiger Phase in Anwesenheit einer katalytisch aktiven Halogenverbindung des fünfwertigen Antimons ist dadurch gekennzeichnet, daß man zur Herstellung von Äthanderivaten der allgemeinen Formel (I)

CF₃CHYZ (I)

worin Y und Z gleich oder verschieden sein können und Y Wasserstoff, Fluor, Chlor oder Brom bedeutet und Z Wasserstoff, Fluor, Chlor oder Brom bedeutet, Äthanderivate der allgemeinen Formel (II)

CHal¹Hal²Hal³CHYZ (II),

worin Y und Z obige Bedeutung besitzen, Hal¹ Halogen, Hal² Halogen und Hal³ Halogen mit Ausnahme von Fluor bedeuten oder Gemische von Äthanderivaten der allgemeinen Formel (II) umsetzt, wobei die Halogenverbindung des fünfwertigen Antimons in Form einer im wesentlichen wasserfreien, katalytisch wirksamen Lösung einer Fluorwasserstoffadditionsverbindung der Formel (III)

H₂F⁺(SbF₆)⁻ (III)

in Fluorwasserstoff vorliegt, wobei in der Reaktionsmischung das Mol-Verhältnis von Fluorwasserstoff zur Fluorwasserstoffadditionsverbindung zwischen 1:1 und 25:1 beträgt, und das gebildete Äthanderivat der allgemeinen Formel (I) aus dem Reaktionsgemisch abtrennt.

Bevorzugt werden Verbindungen der Formel II eingesetzt, worin Hal³ Chlor und Hal¹ und Hal² Fluor oder Chlor bedeuten. Y und Z bedeuten vorzugsweise Chlor.

Die als Ausgangsverbindungen eingesetzten Äthanderivate sind bekannt und können nach bekannten Verfahren hergestellt werden. Es können auch Gemische verwendet werden.

Als Verbindungen der Formel (I), die erfindungsgemäß hergestellt werden können, seien beispielsweise erwähnt:
CF₃CH₃ , CF₃CH₂Cl, CF₃CH₂Br, CF₃CHCl₂, CF₃CHBrCl und CF₃CHBr₂. Besonders gut geeignet ist das Verfahren zur Herstellung von CF₃CH₂Cl und CF₃CHCl₂, insbesondere zur Herstellung von CF₃CHCl₂.

Man geht aus von Verbindungen der allgemeinen Formel (II), wobei der Molekülteil -CHXY in den Verbindungen der Formel (I) und der Formel (II) jeweils die gleiche Bedeutung besitzt.

Beispielsweise kann man zur Herstellung von CF₃CH₂Cl ausgehen von CBrCl₂CH₂Cl, CF₂BrCH₂Cl oder bevorzugt von CCl₃CH₂Cl, CFCl₂CH₂Cl, CF₂ClCH₂Cl oder Gemischen dieser Verbindungen.

Zur Herstellung von CF₃CHCl₂ kann man beispielsweise ausgehen von CBrCl₂CHCl₂, CF₂BrCHCl₂ oder bevorzugt von CCl₃CHCl₂, CFCl₂CHCl₂, CF₂ClCHCl₂ oder Gemischen dieser Verbindungen.

Gewünschtenfalls kann als Äthanderivat ein eine Verbindung der Formel (IIa)

CFHal²Hal³CHYZ (IIa)

worin Hal², Hal³, Y und Z obige Bedeutung besitzen, enthaltendes Gemisch eingesetzt werden, welches in situ durch Umsetzen einer Äthenverbindung der allgemeinen Formel (IV)

CHal²Hal³=CYZ (IV)

worin Hal², Hal³, Y und Z obige Bedeutung besitzen, mit im wesentlichen wasserfreiem Fluorwasserstoff in flüssiger Phase in Gegenwart der katalytisch wirksamen Lösung hergestellt wird.

Das erfindungsgemäße Verfahren wird mit im wesentlichen wasserfreien Fluorwasserstoff durchgeführt. Hierunter wird Fluorwasserstoff mit einem Gehalt von weniger als 0,1 Gew.-%, vorzugsweise weniger als 0,01 Gew.-% Wasser verstanden. Wenn im folgenden von Fluorwasserstoff die Rede ist, wird darunter immer im wesentlichen wasserfreier Fluorwasserstoff verstanden.

Zweckmäßig führt man das Äthanderivat und gegebenenfalls weiteren Fluorwasserstoff in die katalytisch wirksame Lösung ein.

Die Erfindung wird nun anhand der Herstellung von CF₃CHCl₂ aus CFCl₂CHCl₂, CF₂ClCHCl₂, CCl₃CHCl₂ oder Gemischen dieser Verbindungen, weiter erläutert.

Im folgenden wird die Herstellung der katalytisch wirksamen Lösung beschrieben.

Es ist bekannt, daß fünffach koordiniertes Antimonpentafluorid in flüssigem Fluorwasserstoff mit H₂F⁺(SbF₆)⁻ im Gleichgewicht steht:

2HF + SbF₅ = H₂F⁺(SbF₆)⁻

Ein derartiges Gleichgewicht existiert nicht nur zwischen Fluorwasserstoff und Antimonpentafluorid, sondern auch zwischen Fluorwasserstoff und SbClₙ·F₅₋ₙ·, worin n' einen Wert von 0 bis 1 darstellt:

2HF + SbClₙ·F₅₋ₙ· = H₂F⁺ (SbClₓF_{y})⁻

Durch einen Überschuß an Fluorwasserstoff kann das Gleichgewicht auf die Seite der Fluorwasserstoffadditionsverbindung verschoben werden.

Die katalytisch wirksame Lösung erzeugt man also zweckmäßig, indem man eine Antimonverbindung der Formel SbClₙF₅₋ₙ, worin n einen Wert von 0 bis 5 besitzt, mit einer zur Bildung einer Lösung der Verbindung der Formel III in Fluorwasserstoff ausreichenden Menge Fluorwasserstoff bei solcher Temperatur und solchem Druck umsetzt, daß das SbClₙF₅₋ₙ im wesentlichen vollständig unter Bildung von H₂F⁺(SbF₆)⁻ umgewandelt wird.

Hierzu bieten sich verschiedene Möglichkeiten.

Besonders einfach läßt sich eine katalytisch wirksame Lösung erzeugen, indem man Antimonpentafluorid mit Fluorwasserstoff umsetzt. Zweckmäßig verwendet man pro Mol Antimonpentafluorid mindestens 2 Mol Fluorwasserstoff. Die katalytisch wirksame Lösung bildet sich dann bereits bei Raumtemperatur. Zweckmäßig arbeitet man bei erhöhtem Druck, um den Fluorwasserstoff in flüssiger Phase zu halten.

Die katalytisch wirksame Lösung kann auch hergestellt werden, indem man Antimonpentahalogenid, beispielsweise 5-fach koordiniertes Antimonchloridfluorid oder vorzugsweise Antimonpentachlorid, mit Fluorwasserstoff bei erhöhter Temperatur und erhöhtem Druck umsetzt. Bei Anwendung von Antimonpentachlorid führt man die Umsetzung vorzugsweise bei Temperaturen zwischen 20 °C und 100 °C und einem Druck von 0,5 bis 8 bar (abs) durch. Der Verlauf der Umsetzung kann beispielsweise anhand des entstehenden Chlorwasserstoff verfolgt werden.

Im Rahmen der vorliegenden Erfindung ist es wichtig, daß das vorhandene Antimonpentahalogenid vorwiegend als Fluorwasserstoffadditionsverbindung vorliegt mit der Formel H₂F⁺(SbClₓF_{y})⁻, worin x und y zusammen 6 ergeben und x einen Wert von 0 und y einen Wert von 6 darstellen, gelöst in überschüssigem Fluorwasserstoff. Das Mol-Verhältnis von Fluorwasserstoff zur Additionsverbindung ist deshalb während der gesamten Umsetzung nicht kleiner als etwa 1:1. Vorteilhafterweise beträgt das Mol-Verhältnis von Fluorwasserstoff zu Additionsverbindung 3:1 bis 10:1. Die Fluorwasserstoffadditionsverbindung ist dann in einer Menge von etwa 33 bis 93 Gew.-%, vorzugsweise von etwa 60 bis 81 Gew.-% in Fluorwasserstoff enthalten.

Bevorzugt führt man die Umsetzung durch, ohne daß 3-wertiges Antimon in der Reaktionsmischung vorhanden ist. Es wird zweckmäßig auch kein 3-wertiges Antimon bei der Herstellung der katalytisch aktiven Lösung verwendet.

Ein zusätzliches Lösungsmittel, beispielsweise ein inerter Halogenkohlenwasserstoff, wird nicht benötigt.

Das erfindungsgemäße Verfahren kann gewünschtenfalls in Anwesenheit zusätzlicher, die Reaktion fördernder Katalysatoren durchgeführt werden. Bevorzuat ist die Verbindung der Formel (III) die einzige katalytisch aktive Komponente.

Zur Herstellung von CF₃CHCl₂ werden nun in einer bevorzugten Ausführungsform in die zuvor hergestellte katalytisch aktive Lösung das Äthan- oder Äthenderivat und gegebenenfalls Fluorwasserstoff eingeleitet. Das Äthan- oder Äthenderivat und Fluorwasserstoff können gleichzeitig, gewünschtenfalls als Gemisch, eingeleitet werden.

Aus der vorstehenden Beschreibung ergibt sich, daß in der Reaktionsmischung immer Fluorwasserstoff neben der Additionsverbindung vorhanden sein soll und daher für die Umsetzung mit der oder den Ausgangsverbindungen nicht verbraucht werden soll. Die eingesetzte Menge an Fluorwasserstoff, die über die nicht an der Umsetzung teilnehmende Menge an Fluorwasserstoff hinausgeht, sollte derart bemessen sein, daß sie mindestens der zum Chlor-Fluor-Austausch, bzw., bei Verwendung von Perchlorethylen als Ausgangsverbindung, der zur Fluorwasserstoffanlagerung und zum Chlor-Fluor-Austausch stöchiometrisch notwendigen Menge entspricht. Bevorzugt entspricht sie einem stöchiometrischen Überschuß. Gute Ergebnisse werden erzielt, wenn Fluorwasserstoff in einer Menge eingesetzt wird, die dem 1,3 bis 2,5-fachen der stöchiometrisch benötigten Menge entspricht.

Gegebenenfalls wird Fluorwasserstoff eingeleitet, so daß in dem Reaktionsgemisch immer ein Mindestverhältnis von freiem HF zur Additionsverbindung der Formel (III) von etwa 1:1, z.B. 1:1 bis 25:1 erhalten bleibt.

Es ist vorteilhaft, für intensive Durchmischung der katalytisch wirksamen Lösung und der zu fluorierenden Verbindung oder des Gemisches von Verbindungen zu sorgen, da sich ein Zweiphasensystem ausbilden kann. Die vorteilhafte, intensive Durchmischung kann durch entsprechende leistungsstarke Rührwerke, durch Eindüsen von Inertgas, beispielsweise Stickstoff, durch Eindüsen von Fluorwasserstoff oder Umwälzen des Reaktionsgemisches mit einer Pumpe über einen statischen Mischer erfolgen.

Die Umsetzung kann batchweise oder kontinuierlich erfolgen. Arbeitet man batchweise, wird die Gesamtmenge an Äthanderivat und Fluorwasserstoff in Gegenwart der katalytischen Lösung in einem Reaktor umgesetzt und das erhaltene Reaktionsgemisch anschließend aufgearbeitet. Die Aufarbeitung kann beispielsweise durch destillative Abtrennung flüchtiger Verbindungen, Auswaschen mitgeführter anorganischer Bestandteile und anschließende Trocknung und Kondensation des Reaktionsprodukts erfolgen. Gewünschtenfalls können etwaig vorhandene Nebenprodukte durch weitere Fraktionierung, beispielsweise eine Destillation, vom gewünschten Produkt abgetrennt werden.

Bevorzugt arbeitet man in kontinuierlicher Verfahrensweise. Hierbei werden dem Reaktor kontinuierlich die zu fluorierende Verbindung bzw. das zu fluorierende Gemisch von Verbindungen und Fluorwasserstoff zugeführt und gleichzeitig eine den zugeführten Ausgangsverbindungen entsprechenden Menge umgesetzter Reaktionsmischung abgeführt. Dabei wird Fluorwasserstoff in einer solchen Menge zugeführt, daß die vorstehend beschriebenen Mindestverhältnisse von Fluorwasserstoff zu Fluorwasserstoffadditionsverbindung der Formel (III) eingehalten werden.

Das aus dem Reaktor abgeführte Reaktionsgemisch wird beispielsweise destillativ aufgetrennt. Im Reaktionsgemisch enthaltene Ausgangsverbindungen verbleiben in der höher siedenden Fraktion und können in dem Reaktor rückgeführt werden. Die flüchtigen Bestandteile können wie oben beschrieben aufgearbeitet werden.

Eine andere Möglichkeit besteht darin, die dampfförmig ausgetriebene Reaktionsmischung zu kondensieren. Es bilden sich hierbei zwei Phasen. Die schwerere Phase enthält das gewünschte fluorierte Reaktionsprodukt. Diese Phase kann von der leichteren Phase abgetrennt und gewünschtenfalls destillativ aufgearbeitet werden. Die leichtere Phase enthält Fluorwasserstoff. Dieser wird zweckmäßigerweise in den Reaktor zurückgepumpt. Nicht kondensierende Bestandteile der Reaktionsmischung, vorwiegend HCl, werden durch einen Gaswäscher geleitet, der mit Wasser gefüllt ist. Die wasserlöslichen Bestandteile, insbesondere Salzsäure und etwaig mitgerissener Fluorwasserstoff, werden hier aus dem Gas ausgewaschen. Zur Entfernung etwaig mitgerissener organischer Bestandteile kann man das von HCl befreite Gas in eine Tiefkühlfalle leiten.

Man arbeitet vorteilhafterweise bei Temperaturen von etwa 80 °C bis 200 °C, vorzugsweise 100 °C bis 150 °C. Bei tieferen Temperaturen erfolgt die Umsetzung sehr langsam und erfordert hohe Verweilzeiten. Bei höheren Temperaturen entstehen zunehmend Nebenprodukte.

Die Reaktion wird bei einem solchen Druck durchgeführt, daß die Umsetzung in der flüssigen Phase erfolgt. Der Druck liegt zweckmäßigerweise zwischen 10 und 20 bar (abs.), vorzugsweise zwischen 10 und 16 bar (abs.). Man kann zwar auch bei höheren Drücken arbeiten, dies erfordert jedoch entsprechend teuere Druckapparaturen.

In einer besonders bevorzugten Ausführungsform des erfindungsgemäßen Verfahrens geht man von CCl₂=CCl₂ aus. Bei der Umsetzung von CCl₂=CCl₂ mit dem Fluorwasserstoff bildet sich intermediär in situ ein Gemisch, das CFCl₂CHCl₂ enthält. Dieses Gemisch wird direkt zu CF₃CHCl₂ weiter umgesetzt.

Zweckmäßig führt man die Umsetzung ausgehend von CCl₂=CCl₂ in zwei Stufen durch. Diese besonders bevorzugte Ausführungsform des erfindungsgemäßen Verfahrens ist dadurch gekennzeichnet, daß man die Umsetzung von Fluorwasserstoff mit CCl₂=CCl₂ unter in-situ-Bildung von CFCl₂CHCl₂ enthaltenden Gemischen bei Temperaturen von 60 bis 150 °C, vorzugsweise 60 bis 120 °C und einem Druck von 10 bis 30 bar (abs.), vorzugsweise 15 bis 25 bar (abs.), und die weitere Umsetzung des CFCl₂CHCl₂ enthaltenden Gemisches mit weiterem Fluorwasserstoff bei Temperaturen von 80 bis 200 °C, vorzugsweise 100 bis 150 °C und einem Druck von 10 bis 20 bar (abs.), vorzugsweise 10 bis 16 bar (abs.) durchführt.

Für die Durchführung dieser besonders bevorzugten Ausführungsform mit zweistufiger Durchführung bieten sich zwei besonders zweckmäßige Ausführungsarten an.

Die eine Ausführungsart eignet sich besonders für die batchweise Herstellung von CF₃CHCl₂. Beide Stufen werden hier zweckmäßig im gleichen Reaktor nacheinander durchgeführt. Man geht dann so vor, daß man die Umsetzung von CCl₂=CCl₂ mit Fluorwasserstoff zunächst unter den Bedingungen der in-situ-Herstellung der CFCl₂CHCl₂ enthaltenden Gemische hinsichtlich Temperatur und Druck durchführt, und, wenn ein wesentlicher Teil, also beispielsweise mehr als 70 Gew.-% des ursprünglich vorhandenen CCl₂=CCl₂, umgesetzt ist, im Reaktor die Bedingungen der Fluorierung hinsichtlich Temperatur und Druck einstellt.

Die andere Ausführungsart eignet sich für die kontinuierliche Durchführung des Verfahrens. Diese Ausführungsart eignet sich ganz besonders gut für die technische Herstellung von CF₃CHCl₂. In dieser Ausführungsart werden die in-situ-Herstellung der CFCl₂CHCl₂ enthaltenden Gemische und deren weitere Umsetzung zu CF₃CHCl₂ räumlich getrennt durchgeführt. Man verwendet hierbei zwei Reaktoren und setzt in beiden Reaktoren die vorstehend beschriebene katalytisch aktive Lösung ein. Auch bei dieser Ausführungsart leitet man zweckmäßig bevorzugt die Reaktionspartner in die katalytisch wirksame Lösung ein.

Im ersten Reaktor wird kontinuierlich zugeführtes CCl₂=CCl₂ mit kontinuierlich zugeführtem Fluorwasserstoff bei für die in-situ-Bildung von CFCl₂CHCl₂ geeignetem Druck und Temperatur umgesetzt. Das gebildete, CFCl₂CHCl₂-haltige Gemisch wird sodann in einen zweiten Reaktor überführt und darin bei für die Bildung des CF₃CHCl₂ geeigneten Druck und Temperatur mit Fluorwasserstoff umgesetzt. Die Überführung der Reaktionsmischung vom 1. in den 2. Reaktor erfolgt zweckmäßigerweise in der Dampfphase, weil dann allenfalls geringe Mengen der katalytisch aktiven Lösung und geringere Mengen an Ausgangsverbindung(en) mitgerissen werden. Die Überführung kann beispielsweise mittels einer rückschlagsicheren Pumpe erfolgen.

Das Reaktionsprodukt stellt ein Gemisch dar, welches Fluorwasserstoff, Chlorwasserstoff, CF₂ClCHCl₂, CF₃CHCl₂ und Verunreinigungen, beispielsweise C₂Cl₃F₃ und C₂Cl₂F₄ enthält. Diese und gegebenenfalls weitere perhalogenierte Nebenprodukte fallen nur in sehr geringer Konzentration an.

Dieses Produktgemisch kann unter Druckentspannung zunächst durch fraktionierte Kondensation und anschließende destillative Aufarbeitung aufgetrennt werden.

Gewünschtenfalls kann man das aus dem zweiten Reaktor ausgetriebene Reaktionsgemisch vor der Aufarbeitung in eine Beruhigungszone überführen. In dieser Beruhigungszone kann man leichtsiedende Bestandteile der organischen Phase, vorwiegend das gewünschte Reaktionsprodukt, gasförmig abtrennen. Die kondensierte hochsiedende Phase enthält Ausgangsverbindungen und mitgerissene Katalysatorprodukte und wird in den Reaktor zurückgeführt. Beispielsweise kann die Beruhigungszone einen Stripper mit aufgesetzter Destillationskolonne umfassen. Hier werden die höher siedenden Bestandteile der Reaktionsmischung durch die Destillationskolonne aus dem Dampfstrom abgetrennt und laufen in den Reaktor zurück.

Es ist auch hier möglich, das dampfförmig ausgetriebene Reaktionsgemisch zunächst zu kondensieren. Es bilden sich dann zwei Phasen. Die schwerere Phase, die das gewünschte Reaktionsprodukt enthält, wird von der leichteren Phase abgetrennt und kann gewünschtenfalls noch destillativ aufgearbeitet werden. Die leichtere Phase enthält Fluorwasserstoff und wird zweckmäßigerweise in den oder die Reaktoren zurückgepumpt.

Nichtkondensierende Bestandteile werden wiederum durch einen Gaswäscher geleitet und gewünschtenfalls, zur Abtrennung etwaig mitgerissener organischer Bestandteile, in eine Tiefkühlfalle geleitet.

Das nach diesem Verfahren produzierte 1.1.1.-Trifluor-2.2.-dichlorethan kann als Alternative zu CFCl₃ als Lösemittel oder Treibmittel verwendet werden. Die teilfluorierten Produkte wie CFCl₂CHCl₂ und CF₂ClCHCl₂ können in das erfindungsgemäße Verfahren rückgeführt werden. Rückgeführt werden auch die anorganischen fluorhaltigen Bestandteile wie Fluorwasserstoff. Der abgetrennte Chlorwasserstoff kann zu Salzsäure aufgearbeitet werden. Sofern man im erfindungsgemäßen Verfahren nach der bevorzugten Ausführungsform von CCl₂=CCl₂ ausgeht, kann es günstig sein, einen Teil des abgetrennten Chlorwasserstoffes in die erste Reaktionsstufe zurückzuführen, da diese einfache Maßnahme zur Erhöhung der Reaktionsgeschwindigkeit und des Umsatzgrads des erfindungsgemäßen Verfahrens beitragen kann.

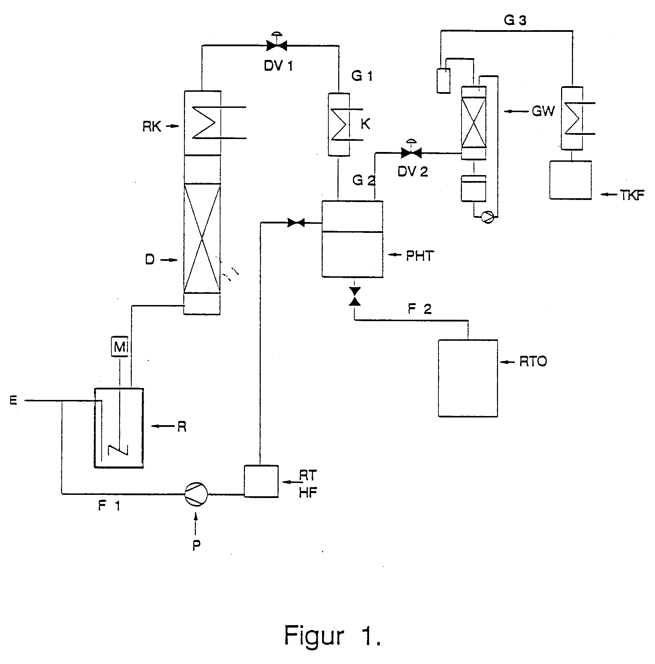

Eine einfache, zweckmäßige Apparatur zur Durchführung des erfindungsgemäßen Verfahrens zeigt die Figur 1. Gewünschte Abwandlungen dieser Vorrichtung kann der Fachmann leicht vornehmen. Beispielsweise ist es ohne weiteres möglich, anstelle des in der Figur 1 gezeigten einstufigen Reaktors einen zweistufigen Reaktor vorzusehen.

Die in Figur 1 gezeigte Apparatur weist einen Reaktor R auf. Dieser Reaktor R sollte zweckmäßigerweise aus einem gegenüber Fluorwasserstoff und Antimonhalogenid resistenten Werkstoff sein. Gut geeignet sind beispielsweise Reaktoren aus Hastelloy-Legierung. Der Reaktor R weist einen Einlaß auf, in den über die Leitung E die Ausgangsmaterialien eingebracht werden können. Weitere Öffnungen, die in des Figur nicht wiedergegeben sind, ermöglichen die Temperaturkontrolle und die Entnahme von Analysenproben. Der Reaktor R weist weiterhin eine durch den Motor M betriebene Rühreinrichtung auf und einen Auslaß für die Reaktionsprodukte. Dieser Auslaß ist mit einer Destillationskolonne D verbunden, die ihrerseits mit einem Rückflußkühler RK verbunden ist. Der Rückflußkühler RK ist durch die Leitung G1 mit einem kühlbaren Kondensator K verbunden. Zwischen dem Rückflußkühler RK und dem Kondensator K ist ein Druckhalteventil DV1 in die Leitung G1 eingebaut.

Der Kondensator K ist mit einem Phasentrenner PHT verbunden. In diesem Phasentrenner PHT bildet sich ein aus zwei Phasen bestehendes Kondensat. Die schwerere Phase enthält die organischen Reaktionsprodukte. Die leichtere Phase besteht im wesentlichen aus Fluorwasserstoff. Diese obere Phase wird in einen HF-Recycle-Tank RTHF überführt und mittels einer Pumpe P durch die Leitung F1 in den Reaktor zurückgeführt.

Die schwerere Phase wird durch die Leitung F2 in einen Rohtank RTO abgelassen und aus diesem gewünschtenfalls destillativ aufgearbeitet.

Nicht kondensierende Gase werden durch die Gasleitung G2 und das Druckhalteventil DV2 in einen Gaswäscher GW entspannt. Hier werden wasserlösliche Besandteile, insbesondere Salzsäure und etwaig mitgerissener Fluorwasserstoff ausgewaschen. Das den Gaswäscher verlassende Restgas wird durch die Leitung G3 in eine Tiefkältefalle TKF geleitet. Hier kondensieren etwaig mitgerissene organische Bestandteile.

Abwandlungen dieser Apparatur kann der Fachmann leicht vornehmen. So kann er beispielsweise anstelle des einstufigen Reaktors R einen zweistufigen Reaktor vorsehen. Gewünschtenfalls kann die Tiefkühlkondensations-Einrichtung mit einer weiteren Destillationskolonne verbunden sein. Diese und weitere Abwandlungen der verwendeten Apparatur liegen im Bereich des Fachwissens.

Das erfindungsgemäße Verfahren bietet besondere Vorteile:
- es erfolgt energiesparend in der flüssigen Phase
- die Ausbeute ist um ein vielfaches höher als bei bislang bekannten Verfahren
- es kann kontinuierlich betrieben werden
- es fallen wenig Nebenprodukte an, insbesondere wenig perhalogenierte Verbindungen
- der Katalysator ist langlebig, die Entsorgungsprobleme sind entsprechend gering
Die folgenden Beispiele sollen das erfindungsgemäße Verfahren weiter erläutern, ohne seinen Schutzumfang einzuschränken.

### Beispiel 1:

### Herstellung von 1.1.1.-Trifluor-2.2.-dichlorethan im batch-Verfahren.

### 1.1. verwendete Apparatur

Als Reaktor wurde ein Autoklav mit einem Innenvolumen von 30 Litern verwendet. Der Autoklav war mit einer Rühreinrichtung sowie mit einem verschließbaren Einlaß zur Zuführung der Ausgängsverbindungen versehen. Er war über einen Auslaß mit einer aufgesetzten Destillationskolonne und einem sich an die Kolonne anschließenden Rückflußkühler versehen.

Der Rückflußkühler war mit einer Gasleitung verbunden, die in einen kühlbaren Kondensator mündete. Der Kondensator war über eine Leitung mit einem Phasentrenner verbunden. Aus dem Phasentrenner führte eine Gasleitung in einen Gaswäscher. Der Gaswäscher war mit einem Tiefkältekondensator verbunden. Ein Druckhalteventil, welches hinter dem Rückflußkühler in die Gasleitung eingebaut war, hielt den Druck im Autoklaven konstant.

Der Phasentrenner war weiterhin mit einem Rohtank verbunden, in welchen die schwerere organische Phase abgelassen werden konnte, sowie mit einem Tank, in welchen die leichtere, Fluorwasserstoff enthaltende Phase abgelassen werden konnte.

Die Destillationskolonne und der Rückflußkühler wurden während der Umsetzung auf 90 °C temperiert. Nicht umgesetzte organische Bestandteile kondensierten hier und liefen in den Reaktor zurück.

### 1.2. Herstellung dar katalytisch aktiven Lösung

In den Autoklaven wurden 11,0 kg (550 Mol) Fluorwasserstoff vorgelegt und 26,3 kg (121,5 Mol) Antimonpentafluorid unter Rühren zugegeben. Es bildete sich eine Lösung von H₂F⁺(SbF₆)⁻ in Fluorwasserstoff.

### 1.3. Fluorierung von Perchlorethylen zu 1.1.1.-Trifluor-2.2.-Dichlorethan

Zu der unter 1.2. beschriebenen katalytisch aktiven Lösung wurden 2,7 kg (135 Mol) Fluorwasserstoff und 5,84 kg (35 Mol) Perchlorethylen zugegeben. Das Reaktionsgemisch wurde 8 Stunden bei 135 °C gerührt. Während der Umsetzung entwickelten sich Gase, im wesentlichen Chlorwasserstoff, welche einen Druckanstieg bewirkten. Der Druck wurde durch entsprechende Einstellung des hinter dem Kühler befindlichen Druckhalteventils auf einen Maximaldruck von etwa 16 bar (abs.) eingeregelt. Sobald der Druck im Reaktionssystem den eingestellten Maximaldruck überschritt, wurden gasförmige Reaktionsprodukte, insbesondere HCl, hinter der Destillationskolonne und dem Rückflußkühler über das Druckhalteventil aus dem Autoklaven abgeleitet. Höher siedende Bestandteile wurden hierbei in der Destillationskolonne und im Rückflußkühler auskondensiert und in den Autoklaven rückgeführt. Die über das Druckhalteventil entweichenden Gase, insbesondere Chlorwasserstoff, wurden im Gaswäscher, der als Absorptionsmittel Wasser enthielt, ausgewaschen.

Nach Beendigung der Gasentwicklung wurde das Reaktionsgemisch auf etwa 90 bis 100 °C abgekühlt und die Destillationskolonne, der Rückflußkühler und das Wasser im Gaswäscher auf etwa 50 °C temperiert. Das Druckhalteventil wurde geöffnet, so daß der Autoklavendruck auf Umgebungsdruck absinken konnte. Unter Umgebungsdruck bei den genannten Bedingungen ist dar Reaktionsprodukt gasförmig. Das aus dem Autoklaven entweichende gasförmige Reaktionsprodukt wurde durch den kühlbaren Kondensator geleitet. Kondensierbare Produkte wurden verflüssigt und im Phasentrenner aufgefangen. Gasförmige Produkte, insbesondere HCl enthaltend, wurden in einen Gaswäscher geleitet.

Aus dem Phasentrenner wurden 4.86 kg eines organischen Reaktionsproduktes in den Rohtank abgelassen. Das Reaktionsprodukt wurde gaschromatographisch analysiert. Es bestand zu etwa 97,3 Gew.-% aus 1.1.1.-Trifluor-2.2.-Dichlorethan neben geringen Mengen an 1.1.-Difluor-1.2.2.-Trichlorethan und 1.1.1.2.-Tetrafluor-2.2.-Dichlorethan. Die Ausbeute betrug 87 %, bezogen auf das eingesetzte Perchlorethylen.

Eine weitere Reinigung des Produkts erfolgte durch Destillation.

### Beispiel 2:

### Herstellung von 1.1.1.-Trifluor-2.2.-Dichlorethan in kontinuierlicher Verfahrensweise

### 2.1. Verwendete Apparatur

Die in diesem Beispiel verwendete Apparatur entsprach der in Beispiel 1.1. beschriebenen Apparatur, als Reaktor wurde diesmal ein 5 l-Druckreaktor aus Hastelloy^{R} C22 (eine fluorwasserstoffresistente Nickellegierung) mit aufgesetzter Trennkolonne und Kühler verwendet. Die Trennkolonne und der Kühler wurden diesmal auf 85 °C temperiert.

### 2.2.Herstellung der katalytisch aktiven Lösung

Im Druckreaktor wurden 5 kg (23,4 Mol) Antimonpentafluorid unter Rühren in 1,7 kg Fluorwasserstoff aufgelöst.

### 2.3. Reaktionsdurchführung

In den auf etwa 125 bis 130 °C temperierten Reaktor wurde kontinuierlich ein Gemisch von Perchlorethylen und Fluorwasserstoff eingeleitet. Bei der Reaktion entwickelten sich Gase, die zu einer Druckerhöhung führten. Das Druckhalteventil wurde so eingeregelt, daß der Druck maximal 16 bar (abs.) betrug. In 7 Stunden wurden 2.270 g Perchlorethylen und 1.500 g Fluorwasserstoff eindosiert.

Die das Reaktionssystem verlassenden Gase wurden kondensiert und im Phasentrenner gesammelt. Man erhielt 2.134 g Rohprodukt. Das Rohprodukt enthielt 75,9 Gew.-% 1.1.1.-Trifluor-2.2.-Dichlorethan, 21,8 Gew.-% 1.1.-Difluor-1.2.2.-Trichlorethan, ferner 1,1 Gew.-% Perchlorethylen und 1,2 Gew.-% perhalogenierte Produkte. Das Gemisch wurde destillativ aufgearbeitet, wobei der Anteil an 1.1.-Difluor-1.2.2.-Trichlorethan zur weiteren Umsetzung in den Reaktor zurückgeführt werden kann.

## Patentansprüche

1. Verfahren zur Herstellung von die Trifluormethylgruppe enthaltenden Äthanderivaten durch Halogen-Fluoraustausch mit im wesentlichen wasserfreiem Fluorwasserstoff in flüssiger Phase in Anwesenheit einer katalytisch aktiven Halogenverbindung des fünfwertigen Antimons, dadurch gekennzeichnet, daß man zur Herstellung von Äthanderivaten der allgemeinen Formel (I)
CF₃CHYZ (I)
worin Y und Z gleich oder verschieden sein können und Y Wasserstoff, Fluor, Chlor oder Brom bedeutet und Z Wasserstoff, Fluor, Chlor oder Brom bedeutet, Äthanderivate der allgemeinen Formel (II)
CHal¹Hal²Hal³CHYZ (II),
worin Y und Z obige Bedeutung besitzen, Hal¹ Halogen, Hal² Halogen und Hal³ Halogen mit Ausnahme von Fluor bedeuten oder Gemische von Äthanderivaten der allgemeinen Formel (II) umsetzt, wobei die Halogenverbindung des fünfwertigen Antimons in Form einer im wesentlichen wasserfreien, katalytisch wirksamen Lösung einer Fluorwasserstoffadditionsverbindung der Formel (III)
H₂F⁺(SbF₆)⁻ (III)
in Fluorwasserstoff vorliegt, wobei in der Reaktionsmischung das Mol-Verhältnis von Fluorwasserstoff zur Fluorwasserstoffadditionsverbindung zwischen 1:1 und 25:1 beträgt, und das gebildete Äthanderivat der allgemeinen Formel (I) aus dem Reaktionsgemisch abtrennt.

2. Verfahren nach Anspruch 1, dadurch gekennzeichnet, daß als Äthanderivat ein eine Verbindung der Formel (IIa)
CFHal²Hal³CHYZ (IIa),
worin Hal², Hal³, Y und Z obige Bedeutung besitzen, enthaltendes Gemisch eingesetzt wird, welches in situ durch Umsetzen einer Äthenverbindung der allgemeinen Formel IV
CHal²Hal³=CYZ (IV),
worin Hal², Hal³, Y und Z obige Bedeutung besitzen, mit im wesentlichen wasserfreiem Fluorwasserstoff in flüssiger Phase in Gegenwart der katalytisch wirksamen Lösung hergestellt wird.

3. Verfahren nach Anspruch 1 oder 2, dadurch gekennzeichnet, daß Y und Z für Chlor stehen.

4. Verfahren nach einem der vorhergehenden Ansprüche, dadurch gekennzeichnet, daß Hal³ Chlor bedeutet und Hal¹ und Hal² Fluor oder Chlor bedeuten.

5. Verfahren nach Anspruch 1, dadurch gekennzeichnet, daß man zur Herstellung von CF₃CHCl₂ ein Äthanderivat aus der Gruppe CCl₃CHCl₂, CFCl₂CHCl₂, CF₂ClCHCl₂ oder deren Gemische einsetzt und das gebildete CF₃CHCl₂ aus dem Reaktionsgemisch abtrennt.

6. Verfahren nach einem der vorhergehenden Ansprüche, dadurch gekennzeichnet, daß man die Umsetzung bei einer Temperatur von 80 °C bis 200 °C, vorzugsweise von 100 °C bis 150 °C, und einem Druck von 10 bis 20 bar (abs.), vorzugsweise von 10 bis 16 bar (abs.) durchführt.

7. Verfahren nach Anspruch 5, dadurch gekennzeichnet, daß als Äthanderivat ein CFCl₂CHCl₂ enthaltendes Gemisch eingesetzt wird, welches in situ durch Umsetzen von CCl₂=CCl₂ mit im wesentlichen wasserfreiem Fluorwasserstoff in flüssiger Phase in Gegenwart der katalytisch wirksamen Lösung hergestellt wird.

8. Verfahren nach Anspruch 7, dadurch gekennzeichnet, daß man die in situ-Umsetzung des CCl₂=CCl₂ zu dem CFCl₂CHCl₂ enthaltenden Gemisch bei einer Temperatur von 60 °C bis 150 °C, vorzugsweise 60 °C bis 120 °C, und einem Druck von 10 bis 30 bar (abs.), vorzugsweise 15 bis 25 bar (abs.) durchführt, und die weitere Umsetzung des CFCl₂CHCl₂ enthaltenden Gemisches bei einer Temperatur von 80 bis 200 °C, vorzugsweise 100 bis 150 °C, und einem Druck von 10 bis 20 bar (abs.), vorzugsweise 10 bis 16 bar (abs.) durchführt.

9. Verfahren nach einem der vorhergehenden Ansprüche, dadurch gekennzeichnet, daß man eine katalytisch wirksame Lösung einsetzt, welche erzeugt wird, indem man eine Antimonverbindung der Formel SbClₙF₅₋ₙ, worin n einen Wert von 0-5 besitzt, mit einer zur Bildung einer Lösung der Verbindung der Formel III gemäß Anspruch 1 in Fluorwasserstoff ausreichenden Menge Fluorwasserstoff bei solcher Temperatur und solchem Druck umsetzt, daß das SbClₙF₅₋ₙ im wesentlichen vollständig unter Bildung von H₂F⁺(SbF₆)⁻ umgewandelt wird.

10. Verfahren nach Anspruch 9, dadurch gekennzeichnet, daß man Antimonpentafluorid mit Fluorwassertoff umsetzt.

11. Verfahren nach Anspruch 9, dadurch gekennzeichnet, daß man zur Herstellung der katalytisch wirksamen Lösung Antimonpentachlorid und Fluorwasserstoff bei einer Temperatur von 20 °C bis 100 °C und einem Druck von 0,5 und 8 bar (abs.) umsetzt.

12. Verfahren nach einem der vorhergehenden Ansprüche, dadurch gekennzeichnet, daß man die dampfförmig ausgetriebene Reaktionsmischung unter Bildung zweier Phasen kondensiert und die schwerere, das gewünschte Fluorierungsprodukt enthaltende Phase von der leichteren Phase abtrennt.

## Claims

1. Process for the preparation of ethane derivatives containing the trifluoromethyl group by halogen-fluorine exchange with essentially anhydrous hydrogen fluoride in liquid phase in the presence of a catalytically active halogen compound of pentavalent antimony, characterised in that for the preparation of ethane derivatives of the general formula (I)
CF₃CHYZ (I),
in which Y and Z may be identical or different and Y is hydrogen, fluorine, chlorine or bromine and Z is hydrogen, fluorine, chlorine or bromine, ethane derivatives of the general formula (II)
CHal¹Hal²Hal³CHYZ (II),
in which Y and Z have the above meaning, Hal¹ is halogen, Hal² is halogen and Hal³ is halogen with the exception of fluorine, or mixtures of ethane derivatives of the general formula (II) are reacted, the halogen compound of pentavalent antimony being present in the form of an essentially anhydrous, catalytically active solution of a hydrogen fluoride addition compound of formula (III)
H₂F⁺(SbF₆)- (III)
in hydrogen fluoride, the molar ratio of hydrogen fluoride to the hydrogen fluoride addition compound in the reaction mixture being between 1:1 and 25:1, and the ethane derivative of the general formula (I) formed is separated off from the reaction mixture.

2. Process according to Claim 1, characterised in that the ethane derivative used is a mixture containing a compound of the formula (IIa)
CFHal²Hal³CHYZ (IIa),
in which Hal², Hal³, Y and Z have the above meaning, which mixture is prepared in situ by reacting an ethene compound of the general formula IV
CHal²Hal³=CYZ (IV)
in which Hal², Hal³, Y and Z have the above meaning, with essentially anhydrous hydrogen fluoride in liquid phase in the presence of the catalytically active solution.

3. Process according to Claim 1 or 2, characterised in that Y and Z represent chlorine.

4. Process according to one of the preceding Claims, characterised in that Hal³ is chlorine and Hal¹ and Hal² are fluorine or chlorine.

5. Process according to Claim 1, characterised in that an ethane derivative from the group comprising CCl₃CHCl₂, CFCl₂CHCl₂ or CF₂ClCHCl₂ or mixtures thereof is used for preparing CF₃CHCl₂, and the CF₃CHCl₂ formed is separated off from the reaction mixture.

6. Process according to one of the preceding Claims, characterised in that the reaction is carried out at a temperature of 80°C to 200°C, preferably of 100°C to 150°C, and a pressure of 10 to 20 bar (abs.), preferably of 10 to 16 bar (abs.).

7. Process according to Claim 5, characterised in that the ethane derivative used is a CFCl₂CHCl₂-containing mixture which is prepared in situ by reacting CCl₂=CCl₂ with essentially anhydrous hydrogen fluoride in liquid phase in the presence of the catalytically active solution.

8. Process according to Claim 7, characterised in that the in-situ reaction of CCl₂=CCl₂ to give the CFCl₂CHCl₂-containing mixture is carried out at a temperature of 60°C to 150°C, preferably 60°C to 120°C, and at a pressure of 10 to 30 bar (abs.), preferably 15 to 25 bar (abs.), and the further reaction of the CFCl₂CHCl₂-containing mixture is carried out at a temperature of 80 to 200°C, preferably 100 to 150°C, and at a pressure of 10 to 20 bar (abs.), preferably 10 to 16 bar (abs.).

9. Process according to one of the preceding claims, characterised in that a catalytically active solution is used which is produced by reacting an antimony compound of the formula SbClₙF₅₋ₙ, in which n has a value from 0-5, with an amount of hydrogen fluoride sufficient for forming a solution of the compound of the formula III according to Claim 1 in hydrogen fluoride at such a temperature and such a pressure that the SbClₙF₅₋ₙ is virtually completely converted with the formation of H₂F⁺(SbF₆)⁻.

10. Process according to Claim 9, characterised in that antimony pentafluoride is reacted with hydrogen fluoride.

11. Process according to Claim 9, characterised in that antimony pentachloride and hydrogen fluoride are reacted at a temperature of 20°C to 100°C and at a pressure of 0.5 and 8 bar (abs.) to prepare the catalytically active solution.

12. Process according to one of the preceding claims, characterised in that the reaction mixture driven out in the form of vapour is condensed, forming two phases, and the heavier phase containing the desired fluorination product is separated off from the lighter phase.

## Revendications

1. Procédé pour préparer des dérivés d'éthane contenant le groupe trifluorométhyle, par échange halogène-fluor avec un acide fluorhydrique essentiellement anhydre, en phase liquide, en présence d'un composé halogéné à activité catalytique de l'antimoine pentavalent, caractérisé en ce que, pour préparer des dérivés d'éthane de formule générale (I)
CF₃CHYZ (I)
dans laquelle Y et Z peuvent être identiques ou différents, et Y est un hydrogène, un fluor, un chlore ou un brome, et Z est un hydrogène, un fluor, un chlore ou un brome, on fait réagir des dérivés d'éthane de formule générale (II)
CHal¹Hal²Hal³CHYZ (II)
dans laquelle Y et Z ont les significations données ci-dessus, Hal¹ est un halogène, Hal² est un halogène et Hal³ est un halogène à l'exception du fluor, ou encore des mélanges de dérivés d'éthane de formule générale (II), le composé halogéné de l'antimoine pentavalent se présentant sous forme d'une solution essentiellement anhydre, à activité catalytique, d'un composé d'addition de l'acide fluorhydrique de formule (III)
H₂F⁺(SbF₆)⁻ (III)
dans l'acide fluorhydrique, auquel cas, dans le mélange réactionnel, le rapport en moles de l'acide fluorhydrique au composé d'addition de l'acide fluorhydrique est compris entre 1:1 et 25:1, et que l'on sépare du mélange réactionnel le dérivé d'éthane ainsi formé, de formule générale (I).

2. Procédé selon la revendication 1, caractérisé en ce qu'on utilise comme dérivé d'éthane un mélange contenant un composé de formule (IIa)
CFHal²Hal³CHYZ (IIa)
dans laquelle Hal², Hal³, Y et Z ont les significations données ci-dessus, mélange que l'on prépare in situ en faisant réagir un composé d'éthène de formule générale (IV)
CHal²Hal³-CYZ (IV)
dans laquelle Hal², Hal³, Y et Z ont les significations données ci-dessus, avec de l'acide fluorhydrique essentiellement anhydre en phase liquide et en présence de la solution à effet catalytique.

3. Procédé selon la revendication 1 ou 2, caractérisé en ce que Y et Z sont le chlore.

4. Procédé selon l'une des revendications précédentes, caractérisé en ce que Hal³ est le chlore, et Hal¹ et Hal² sont le fluor ou le chlore.

5. Procédé selon la revendication 1, caractérisé en ce que, pour préparer le CF₃CHCl₂, on utilise un dérivé d'éthane choisi parmi l'ensemble comprenant CCl₃CHCl₂, CFCl₂CHCl₂, CF₂ClCHCl₂ ou leurs mélanges, et qu'on sépare du mélange réactionnel le CF₃CHCl₂ formé.

6. Procédé selon l'une des revendications précédentes, caractérisé en ce qu'on met en oeuvre la réaction à une température de 80 à 200°C et de préférence de 100 à 150°C sous une pression absolue de 10 à 20 bar et de préférence de 10 à 16 bar.

7. Procédé selon la revendication 5, caractérisé en ce qu'on utilise comme dérivé d'éthane un mélange contenant du CFCl₂CHCl₂, mélange que l'on prépare in situ par réaction de CCl₂=CCl₂ avec de l'acide fluorhydrique essentiellement anhydre, en phase liquide et en présence de la solution à effet catalytique.

8. Procédé selon la revendication 7, caractérisé en ce que la réaction in situ du CCl₂=CCl₂ pour donner le mélange contenant du CFCl₂CHCl₂ est mise en oeuvre à une température de 60 à 150°C et de préférence de 60 à 120°C, sous une pression absolue de 10 à 30 bar et de préférence de 15 à 25 bar, et que l'on met en oeuvre la suite de la réaction du mélange contenant du CFCl₂CHCl₂ à une température de 80 à 200°C et de préférence de 100 à 150°C et sous une pression absolue de 10 à 20 bar et de préférence de 10 à 16 bar.

9. Procédé selon l'une des revendications précédentes, caractérisé en ce qu'on utilise une solution à effet catalytique, que l'on prépare en faisant réagir un composé de l'antimoine de formule SbClₙF₅₋ₙ dans laquelle n a une valeur de 0 à 5, avec une quantité d'acide fluorhydrique suffisante pour former une solution du composé de formule III selon la revendication 1 dans l'acide fluorhydrique, à une température et sous une pression telles que le SbClₙF₅₋ₙ soit converti pratiquement complètement avec formation de H₂F⁺(SbF₆)⁻.

10. Procédé selon la revendication 9, caractérisé en ce qu'on fait réagir le pentafluorure d'antimoine avec de l'acide fluorhydrique.

11. Procédé selon la revendication 9, caractérisé en ce que, pour préparer la solution à effet catalytique, on fait réagir le pentachlorure d'antimoine et l'acide fluorhydrique à une température de 20 à 100°C et sous une pression absolue de 0,5 à 8 bar.

12. Procédé selon l'une des revendications précédentes, caractérisé en ce qu'on condense le mélange réactionnel expulsé sous forme vapeur, avec formation de deux phases, et que l'on sépare de la phase légère la phase lourde, qui contient le produit recherché de fluoration.
